# EUROPÄISCHE PATENTSCHRIFT

(11) **EP 1 345 879 B1**
(45) Veröffentlichungstag und Bekanntmachung des Hinweises auf die Patenterteilung: **21.09.2005**
(21) Anmeldenummer: 01999549.7
(22) Anmeldetag: 26.11.2001
(51) Int. Cl.: C07C 45/86, C07C 47/228, C11B 9/00, A61K 7/46, A23L 1/00

(54) **VERFAHREN ZUR STABILISIERUNG VON PHENYLACETALDEHYD**
METHOD FOR STABILISING PHENYLACETALDEHYDE
PROCEDE DE STABILISATION DE PHENYLACETYLALDEHYDE

(30) Priorität: 07.12.2000 DE 10060755
(43) Veröffentlichungstag der Anmeldung: 24.09.2003
(73) Patentinhaber: Symrise GmbH & Co. KG, 37603 Holzminden (DE)
(72) Erfinder: KUHN, Walter, 37603 Holzminden (DE)
(74) Vertreter: Eisenführ, Speiser & Partner
(86) Internationale Anmeldenummer: PCT/EP2001/013710
(87) Internationale Veröffentlichungsnummer: WO 2002/046135

(56) Entgegenhaltungen:
- DE-A- 19 735 332
- US-A- 2 280 311
- DATABASE WPI Section Ch, Week 199252 Derwent Publications Ltd., London, GB; Class D23, AN 1992-431227 XP002192179 & SU 1 705 273 A (CHEM PHYS INST), 15. Januar 1992 (1992-01-15) in der Anmeldung erwähnt

## Beschreibung

Die vorliegende Erfindung betrifft ein Verfahren zur Stabilisierung von Phenylacetaldeyhd gegen Polymerisation und Autokondensation.

Phenylacetaldehyd kommt in etherischen Ölen vor und ist flüchtiger Bestandteil von Nahrungsmitteln. Sein Geruch ist der Hyacinthe ähnlich. Phenylacetaldehyd kann durch Umlagerung von Styroloxid hergestellt werden und findet in Riechstoffkompositionen und Aromen Anwendung; K. Bauer, A. Garbe, Common Fragance and Flavor Materials, S. 68, VCH, Weinheim, 1985; S. Steffen Arctander, Perfume and Flavor Chemicals, No. 2470, Montclair, New Jersey, USA 1969.

Phenylacetaldehyd neigt aufgrund der großen Reaktivität zur Polymerisation und Autokondensation. Bei der Polymerisation bildet sich vorwiegend 2,4,6-Tribenzyl-1,3,5-trioxan. Die Trimerisierung wird durch den Einfluss chemischer Substanzen wie Chlor oder Brom, Phosphorpentoxid, Schwefelsäure, Schwefelwasserstoff, Chlorwasserstoff, Fluorwasserstoff, Bortrifluorid, Aluminiumchlorid, Eisenchlorid oder Zinkchlorid katalysiert. Unter dem Einfluss acider Verbindungen setzt die Polymerisation von Phenylacetaldehyd spontan ein. Darüber hinaus begünstigen tiefe Temperaturen ab etwa 0°C oder UV-Licht die Polymerisation.

Ein weiteres Problem stellt die Neigung von Phenylacetaldehyd dar, unter Einwirkung alkalischer Substanzen der Aldolkondensation zu unterliegen.

Es ist bekannt, dass die Polymerisations- und Autokondensationsreaktionen bei Aldehyden durch Zusatz bestimmter Stoffe unterbunden werden können. Bei der Verarbeitung und dem Einsatz von Phenylacetaldehyd als Riech- und Aromastoff dürfen die zugesetzten chemischen Substanzen organoleptisch nicht stören und müssen nach dem Lebensmittelgesetz zulässig sein.

Aus der DE-A 29 05 267 sowie der DE-A 29 17 789 ist es bekannt, dass man aliphatische Aldehyde durch den Zusatz von Triethanolamin oder Dimethylethanolamin gegen Polymerisation und Autokondensation stabilisieren kann. Nachteilig an diesen Stabilisatoren ist jedoch, dass sie in Nahrungsmittel nicht erwünscht sind und sich nur mit einem erheblichen destillativen Aufwand wieder von den Aldehyden abtrennen lassen.

In JP 45 012282 B4 und DE A 19757531 wird die Stabilisierung von aliphatischen Aldehyden mit Alkalimetallhydroxiden, Erdalkalimetallhydroxiden, Alkalimetallcarbonaten, Erdalkalimetallcarbonaten oder Erdalkalimetallcarboxylaten beschrieben. Nachteilig an diesem Verfahren ist, dass die zugesetzten Stoffe sich nicht vollständig auflösen und dispergieren. Eine gleichmäßige Verteilung der alkalischen Substanz im gesamten Aldehydvolumen ist schwer zu erreichen.

In SU A 1705273 wird die Stabilisierung von Phenylacetaldehyd durch die Kombination von drei Substanzklassen beschrieben; i.e
1. ein Radikalfänger wie alpha-Tocopherol oder Butylhydroxyanisol
2. ein Diphenylamin-Derivat wie Dimethylbis(p-phenylaminophenoxy)silan und
3. eine Hydroxysäure wie Weinsäure, Ascorbinsäure oder Zitronensäure.

Nachteilig an diesem Verfahren ist der umfangreiche Einsatz an Stoffen. Des weiteren sind die beschriebenen Diphenylaminderivate in Lebensmitteln nicht zulässig.

Aus der DE 197 35 332 A1 ist bekannt, aliphatische Aldehyde zu stabilisieren, indem diese auf einen Wassergehalt > 300 ppm gebracht und ihnen eine organische, gesättigte, Metallkomplexe bildende Säure zugesetzt wird.

Aus der US 2,280,331 wiederum ist bekannt, aromatische Aldehyde durch Thioharnstoff oder dessen Derivate zu stabilisieren.

Aufgabe der vorliegenden Erfindung ist es demgemäss, ein Verfahren zur Stabilisierung von Phenylacetaldehyd durch Zusatz von einem oder mehreren Additiven zur Verfügung zu stellen, das die vorgenannten Nachteile nicht aufweist.

Es wurde ein Verfahren zur Stabilisierung von Phenylacetaldehyd durch alleinigen Zusatz eines oder mehrerer Additive gefunden, das dadurch gekennzeichnet ist, dass die Additive aus einer oder mehreren mehrwertigen Carbonsäuren bestehen.

Erfindungsgemäß werden die mehrwertigen Carbonsäuren in Mengen von 10 bis 1000 ppm eingesetzt. Besonders bevorzugt sind 100 bis 300 ppm.

Als mehrwertige Carbonsäuren kommen insbesondere Oxalsäure, Malonsäure, Bernsteinsäure, Glutarsäure, Adipinsäure, Pimelinsäure, Korksäure, Zitronensäure, Maleinsäure oder Fumarsäure in Betracht.

Die in Betracht kommenden Carbonsäuren sind im Lebensmittelbereich unbedenklich. Demgemäss ist es nicht mehr erforderlich unerwünschte Stoffe mit erheblichem Aufwand wieder abzutrennen. Ebenso ist vorteilhaft, dass die beschriebenen Carbonsäuren vollständig löslich und dispergierbar sind. Dadurch ist eine gleichmäßige Verteilung in dem Aldehydvolumen gewährleistet. Die Folge ist eine optimale Stabilisierung der vorhandenen Phenylacetaldehyde. Überraschend ist, dass durch den alleinigen Zusatz von mehrwertigen Carbonsäuren, d.h. ohne Verwendung weiterer Zusatzstoffe, die beschriebenen Vorteile erreicht werden. Damit stellt sich das erfindungsgemäße Verfahren auch als sehr einfach und preisgünstig dar.

Gegenstand der Erfindung ist eine Phenylacetaldehyd zubereitung dadurch gekennzeichnet, dass sie aus Phenylacetaldehyd und aus einer oder mehreren mehrwertigen

Carbonsäuren als alleiniges Additiv besteht.

Ferner ist Gegenstand der Erfindung die Verwendung des stabilisierten Phenylacetaldehyds zur Herstellung von Riechstoffkompositionen und Aromen.

### Beispiele

### Beispiel 1

Zu frisch destilliertem Phenylacetaldehyd wurden 200 ppm Oxalsäure bzw. 200 ppm Zitronensäure zugesetzt. Diese Proben wurden bei 20°C und 40°C gelagert. Die Abnahme des Phenylacetaldehyd-Gehaltes wurde mit der Carbonyl-Zahl verfolgt:

| | Carbonylzahl bei 20°C nach 28 Tagen |
|---|---|
| Phenylacetaldehyd ohne Zusatz | 93.2 % |
| Phenylacetaldehyd mit Zitronensäure | 99.2 % |
| Phenylacetaldehyd mit Oxalsäure | 99.4 % |

| | Carbonylzahl bei 40°C nach 28 Tagen |
|---|---|
| Phenylacetaldehyd ohne Zusatz | 81.9 % |
| Phenylacetaldehyd mit Zitronensäure | 97.0 % |
| Phenylacetaldehyd mit Oxalsäure | 98.3 % |

Das erfindungsgemäße Verfahren kann ausgeführt werden, indem der Stabilisator vorgelegt wird und Phenylacetaldehyd zugegeben wird oder der Stabilisator zum Phenylacetaldehyd zugegeben wird. Rühren ist nicht erforderlich.

### Beispiel 2

Alpha-Tocopherol ist ein natürliches Antioxidans, das im Lebensmittelbereich eingesetzt wird.

| | Carbonylzahl bei 20 Grad C nach 42 Tagen |
|---|---|
| Phenylacetaldehyd ohne Zusatz | 82.5 % |
| Phenylacetaldehyd mit 100 ppm Zitronensäure | 99.3 % |
| Phenylacetaldehyd mit 100 ppm alpha-Tocopherol | 86.8 % |
| Phenylacetaldehyd mit 300 ppm alpha-Tocopherol | 86.4 % |
| Phenylacetaldehyd mit 500 ppm alpha-Tocopherol | 87.0 % |

## Patentansprüche

1. Phenylacetaldehyd-Zubereitung, **dadurch gekennzeichnet, dass** die Zubereitung besteht aus Phenylacetaldehyd und einer oder mehreren mehrwertigen Carbonsäuren.

2. Phenylacetaldehyd-Zubereitung nach Anspruch 1, **dadurch gekennzeichnet, dass** die Konzentration einer mehrwertigen Carbonsäure des Additivs 10 bis 1000 ppm bezogen auf die Gesamtzubereitung beträgt.

3. Phenylacetaldehyd-Zübereitung nach einem der Ansprüche 1 bis 2, **dadurch gekennzeichnet, dass** die eine oder mehrere mehrwertigen Carbonsäuren ausgewählt sind aus der Gruppe bestehend aus Oxalsäure, Malonsäure, Bernsteinsäure, Glutarsäure, Adipinsäure, Pimelinsäure, Korksäure, Zitronensäure, Maleinsäure und Fumarsäure.

4. Verwendung einer Phenylacetaldehyd-Zubereitung nach einem der Ansprüche 1 bis 3 zum Herstellen von Riechstoffkompositionen oder Aromen.

5. Verwendung einer oder mehrerer mehrwertiger Carbonsäuren als alleiniges Additiv zum Stabilisieren von Phenylacetaldehyd.

6. Verfahren zum Stabilisieren von Phenylacetaldehyd durch alleinigen Zusatz eines Additivs bestehend aus einer oder mehreren mehrwertigen Carbonsäuren.

7. Verfahren nach Anspruch 6, **dadurch gekennzeichnet, dass** eine mehrwertige Carbonsäure vorgelegt und anschließend Phenylacetaldehyd zugegeben wird.

8. Verfahren nach Anspruch 6, **dadurch gekennzeichnet, dass** eine mehrwertige Carbonsäure dem Phenylacetaldehyd zugegeben wird.

9. Verfahren nach einem der Ansprüche 6 bis 8, **dadurch gekennzeichnet, dass** die Konzentration einer mehrwertigen Carbonsäure des Additivs 10 bis 1000 ppm bezogen auf die Gesarritzubereitung beträgt.

10. Verfahren nach einem der Ansprüche 6 bis 9, **dadurch gekennzeichnet, dass** die eine oder mehrere mehrwertigen Carbonsäuren ausgewählt sind aus der Gruppe bestehend aus Oxalsäure, Malonsäure, Bemsteinsäure, Glutarsäure, Adipinsäure, Pimelinsäure, Korksäure, Zitronensäure, Maleinsäure und Fumarsäure.

## Claims

1. Phenylacetaldehyde preparation, **characterized in that** it consists of phenylacetaldehyde and one or more polybasic carboxylic acids.

2. Phenylacetaldehyde preparation according to Claim 1, **characterized in that** the concentration of a polybasic carboxylic acid constituting the additive is 10 to 1000 ppm, based on the total preparation.

3. Phenylacetaldehyde preparation according to Claim 1 or 2, **characterized in that** the one or more polybasic carboxylic acids are selected from the group comprising oxalic acid, malonic acid, succinic acid, glutaric acid, adipic acid, pimelic acid, suberic acid, citric acid, maleic acid and fumaric acid.

4. Use of a phenylacetaldehyde preparation according to one of Claims 1 to 3 for the production of perfume compositions or flavourings.

5. Use of one or more polybasic carboxylic acids as the only additive for stabilizing phenylacetaldehyde.

6. Process for the stabilization of phenylacetaldehyde by the addition only of an additive consisting of one or more polybasic carboxylic acids.

7. Process according to Claim 6, **characterized in that** a polybasic carboxylic acid is taken and phenylacetaldehyde is then added.

8. Process according to Claim 6, **characterized in that** a polybasic carboxylic acid is added to the phenylacetaldehyde.

9. Process according to one of Claims 6 to 8, **characterized in that** the concentration of a polybasic carboxylic acid constituting the additive is 10 to 1000 ppm, based on the total preparation.

10. Process according to one of Claims 6 to 9, **characterized in that** the one or more polybasic carboxylic acids are selected from the group comprising oxalic acid, malonic acid, succinic acid, glutaric acid, adipic acid, pimelic acid, suberic acid, citric acid, maleic acid and fumaric acid.

## Revendications

1. Composition de phénylacétaldéhyde, **caractérisée en ce que** la composition se compose de phénylacétaldéhyde et d'un ou de plusieurs acides carboxyliques polyvalents.

2. Composition de phénylacétaldéhyde selon la revendication 1, **caractérisé en ce que** la concentration d'un acide carboxylique polyvalent de l'additif va de 10 à 1000 ppm par rapport à la composition totale.

3. Composition de phénylacétaldéhyde selon l'une des revendications 1 à 2, **caractérisée en ce que** les un ou plusieurs acides carboxyliques polyvalents sont choisis dans le groupe constitué de l'acide oxalique, l'acide malonique, l'acide succinique, l'acide glutarique, l'acide adipique, l'acide pimélique, l'acide subérique, l'acide citrique, l'acide maléique et l'acide fumarique.

4. Utilisation d'une composition de phénylacétaldéhyde selon l'une des revendications 1 à 3 pour fabriquer des compositions odorantes ou arômes.

5. Utilisation d'un ou de plusieurs acides carboxyliques polyvalents à titre d'additif unique pour stabiliser le phénylacétaldéhyde.

6. Procédé de stabilisation du phénylacétaldéhyde par addition unique d'un additif constitué d'un ou de plusieurs acides carboxyliques polyvalents.

7. Procédé selon la revendication 6, **caractérisé en ce que** l'on place un acide carboxylique polyvalent et que l'on ajoute ensuite du phénylacétaldéhyde.

8. Procédé selon la revendication 6, **caractérisé en ce qu'**un acide carboxylique polyvalent est ajouté au phénylacétaldéhyde.

9. Procédé selon l'une des revendications 6 à 8, **caractérisé en ce que** la concentration d'un acide carboxylique polyvalent de l'additif va de 10 à 1000 ppm par rapport à la composition totale.

10. Procédé selon l'une des revendications 6 à 9, **caractérisé en ce que** les un ou plusieurs acides carboxyliques polyvalents sont choisis dans le groupe constitué de l'acide oxalique, l'acide malonique, l'acide succinique, l'acide glutarique, l'acide adipique, l'acide pimélique, l'acide subérique, l'acide citrique, l'acide maléique et l'acide fumarique.
